# EUROPEAN PATENT SPECIFICATION

(11) **EP 2 582 461 B1**
(45) Date of publication and mention of the grant of the patent: **13.08.2014**
(21) Application number: 11729179.9
(22) Date of filing: 21.06.2011
(51) Int. Cl.: B03C 1/033, C12Q 1/68, G01N 33/543, G01N 27/72, G01R 33/12

(54) **METHOD FOR DETERMINING ONE OR MORE CHARACTERIZING FEATURES OF A MACROMOLECULE AND AN APPARATUS FOR CARRYING OUT SAID METHOD**
VERFAHREN ZUR BESTIMMUNG VON EINEM ODER MEHREREN CHARAKTERISIERENDEN MERKMALEN EINES MAKROMOLEKÜLS UND VORRICHTUNG ZUR AUSFÜHRUNG DIESES VERFAHRENS
PROCÉDÉ DE DÉTERMINATION D'UNE OU PLUSIEURS PROPRIÉTÉS CARACTÉRISTIQUES D'UNE MACROMOLÉCULE ET APPAREIL POUR RÉALISER LEDIT PROCÉDÉ

(30) Priority: 21.06.2010 NL 2004928
(43) Date of publication of application: 24.04.2013
(73) Proprietor: Technische Universiteit Delft, 2628 CN Delft (NL)
(72) Inventor: DEKKER, Nynke, 2316 ZT Delft (NL); KERSSEMAKERS, Jacob, 1018 MX Amsterdam (NL); LIPFERT, Jan, 2593 PK Den Haag (NL)
(74) Representative: Zeestraten, Albertus W. J.
(86) International application number: PCT/NL2011/050446
(87) International publication number: WO 2011/162603

(56) References cited:
- WO-A1-2005/111596
- US-A1- 2003 166 262
- US-B1- 7 474 184
- CELEDON ALFREDO ET AL: "Magnetic tweezers measurement of single molecule torque.", NANO LETTERS APR 2009 LNKD- PUBMED:19301859, vol. 9, no. 4, April 2009 (2009-04), pages 1720-1725, XP002622188, ISSN: 1530-6992 cited in the application

## Description

The present invention relates to a method for determining one or more characterizing features of a macromolecule. The invention further relates to an apparatus for carrying out said method.

Such a method and apparatus are known from Celedon et al (Magnetic tweezers measurement of single molecule torque, Nano Letters, 9 (2009), 1720-1725) which discloses a method for measuring the torque exerted by a single macromolecule on a bead using a vertically arranged cylindrical magnet and a probe attached to the macromolecule. The probe consists of a Ni-Pt nanorod with a ferromagnetic Ni segment, coupled to a 1 µm superparamagnetic bead. The nanorod functions as an asymmetry introducing connecting moiety. The coupling between the Ni-segment and the superparamagnetic bead is a result of the magnetic attraction between them. This means that the coupling between the Ni-segment and the superparamagnetic bead can be induced by self-assembly. The probe is prepared separately using electrochemical template techniques.

The magnetic field at the position of the macromolecule is directed predominantly vertically, instead of horizontally as in conventional magnetic tweezers. Due to the vertically directed magnetic field, the probe experiences a low torsional stiffness of the angular trap. This allows for the determination of angular deviations in the orientation of the probe. In conventional magnetic tweezers methods, the orientation of the paramagnetic marker in the horizontal plane is essentially determined by the magnetic field, since the influence of any torque influenced by the attached macromolecule (in the case of Celedon et al, doublestranded DNA (dsDNA)) is too small to be measured. In the Celedon measuring device the substrate to which the macromolecule is attached is rotated in order to induce turns in the macromolecule and to measure the resulting torque and other features of the macromolecule being tested. It is said that this configuration allows a torque resolution of <1 pN.nm.

A disadvantage of the method according to Celedon is that the preparation of the macromolecule-nanorod-bead-assembly is cumbersome, laborious and time-consuming.

Another disadvantage is that special equipment and methods in the form of an electroplating apparatus with multiple electroplating baths are required to prepare the nanorods.

Yet another disadvantage is that in order to apply torque to the DNA molecule under study the glass substrate (i.e. the flow cell) has to be rotated around the magnetic axis. These rotations induce mechanical vibrations that are highly undesirable in sensitive single-molecule experiments.

These rotations induce mechanical vibrations that are highly undesirable in sensitive single-molecule experiments.

Still another disadvantage is the construct complexity is expected to imply a low yield, or density of suitable constructs visible to the microscope. This opposes a well-established advantage of magnetic tweezers, namely the possibility of viewing and measuring many macromolecular assemblies at the same time.

A further disadvantage is the very non-spherical nature of the assembly, which complicates tracking and force calibration procedures that are normally based on the assumption of spherical or near-spherical objects.

The apparatus as described by Celedon et al. is limited to stretching forces of 1.5 pN or less and has a resolution in the z-direction of approximately 50 nm.

US2003/166262A discloses DNA molecules with one end anchored to a fixed surface and at its other end to a paramagnetic bead which serves as a marker; a magnetic force is applied to the beads resulting in rotation and/or stretching of the bead and molecule. This document provides an optical magnification means and a camera for monitoring the movement of said bead.

US7474184 discloses hybrid magnetic tweezers comprising inter alia a ferromagnetic pole and at least two blocks of permanent magnetic material on opposite sides and adjacent to the ferromagnetic pole. It is explained that the magnetization orientations, i.e. the magnetic axes of the blocks of permanent magnetic material are oriented orthogonal to the height of the ferromagnetic pole.

WO2005/111596 mentions the measurement of the magnetic particle rotation. However, what is measured in WO2005/111596, are rotation frequencies, wherein the rotation frequency provides information about the binding state of a certain type of particle in order to be able to distinguish between different types of binding of a magnetic nanoparticle label to the surface of another entity. No mention is made of measurement of determining a starting position or measuring position of a single macromolecule.

It is an object of the present invention to provide a measuring method which shows the above drawbacks to a lesser extent or not at all.

More specifically, it is an object of the present invention to provide a measuring method which is easier to perform because of a less complex preparation of the probe and the compatibility of this preparation with well-defined, commercially available particles.

Another object of the invention is to improve the accuracy of the measuring results.

A further object is to provide a measuring method which allows for the measurement of twist of the macromolecule.

A further object is to stay as close as possible to well-established pulling force calibration procedures.

It is yet another object of the invention to provide an apparatus for carrying out the method according to the invention, in particular for measuring torque and/or twist in a macromolecule.

One or more of the above objects are achieved with a method for determining one or more characterizing features of a macromolecule according to the invention, said method comprising the steps of:
a) providing a first end of the macromolecule with a paramagnetic marker, thereby forming a macromolecule-paramagnetic marker-assembly;
b) tethering at least one other end of the macromolecule of the macromolecule-paramagnetic marker-assembly to one or more tethering points of a holding means for holding said other end of the macromolecule, said tethering points being situated in a x,y-plane such that the macromolecule-paramagnetic marker-assembly is arranged substantially in the z-direction perpendicular to the x,y-plane, between a main magnet arranged substantially perpendicular to the x,y-plane and the surface of the holding means, wherein the poles of the main magnet are arranged along the z-direction and wherein the main magnet generates a magnetic field directed substantially in the z-direction at the position of the macromolecule-paramagnetic marker-assembly;
c) determining a starting position of the paramagnetic marker of the macro-molecule-paramagnetic marker-assembly in a magnetic field generated by the main magnet;
d) rotating the main magnet around its magnetic axis a predetermined number of times, thereby causing the paramagnetic marker to rotate around the magnetic axis of the main magnet, to a measuring position;
e) determining the measuring position of the paramagnetic marker of the macromolecule-paramagnetic marker-assembly;
f) calculating one or more characterizing features from said measuring position;
wherein in step a) the paramagnetic marker is directly attached to the macromolecule, and prior to step c), an auxiliary magnetic field is generated by means of a side magnet.

According to the invention, a paramagnetic marker is directly attached to the macromolecule, which allows for the application of forces and torques to the macromolecule on the one hand, and detection and rotation of the macromolecule on the other hand.

Said torques are applied by rotating the main magnet. The advantage of rotating the main magnet and not the holding means as is done in Celedon et al is that no mechanical vibrations are introduced that would seriously deteriorate the accuracy of the single-molecule measurements described in the present application.

Applicants found that the macromolecule-paramagnetic marker-assembly allows for the determination of the starting position and measuring position of the paramagnetic marker in a weak angular trap at a large range of pulling forces (e.g. 0.1 pN-10 pN). Typically the method is performed at a stiffness of the angular trap *kᵣₒₜ* of 100-500 pN.nm/rad, resulting in fluctuations of > 5° around the equilibrium. This allows to directly observe the shift in equilibrium angle induced by the torque exerted by the macromolecule.

More specifically the paramagnetic markers held in the magnetic field experience a constant upward force *F* due to the gradient of the magnetic field, which is balanced by the restoring force of the macromolecule tether. In addition, the rotation of the marker about the z-axis (along the direction of the stretched macromolecule) is constrained by the magnetic field. The stiffness of the torsional trap *kᵣₒₜ* can be calibrated by observing the rotational thermal fluctuations (δθ²) of the marker and applying the equipartition theorem *kᵣₒₜ*= *k_{B}T*/(δθ²) where *k_{B}* is the Boltzmann constant and *T* the temperature. If the macromolecule tether is over- or underwound by rotating the magnets, the macromolecule will exert a restoring torque *T_{DNA}* on the paramagnetic marker. After calibrating the trap stiffness and the equilibrium angle θ₀ of the trap for a torsionally relaxed macromolecule, *T_{Macromolecule}* can be determined from the shift in the average angle (θ- θ₀) away from this equilibrium. *T_{Macromolecule} = kᵣₒₜ*(θ- θ₀), following said rotation of the magnetic field. it will not be found in nature. Examples of natural polymers in an unnatural configuration are the DNA origami configurations presented by Douglas et al (Nature, 459 (2009), 414-418).

One of the advantages of magnetic tweezers that also applies to the method according to the invention is that a magnetic tweezers setup allows for the parallel measurement of multiple macromolecules. Advantageously, with the method according to the invention, one can measure an array of macromolecules with paramagnetic markers attached thereto, e.g. a membrane comprising macromolecules, on which paramagnetic markers have been attached to several of the macromolecules.

Typically, the method according to the invention is applied to a macromolecule comprising two or more intertwining strands to which a paramagnetic marker can be attached, such as DNA or RNA. In the context of this application, the macromolecule may also be a complex of two or more macromolecules, e.g. a polynucleotide moiety complexed with one or more protein moieties such as chromatin. Preferably, the macromolecule is a polynucleotide moiety or a polynucleotide moiety - protein complex. Polynucleotide-protein complexes play an important role in cell processes, e.g. in processes involved in the replication, transcription and repair of DNA or RNA. In these types of processes, the intertwining of DNA or RNA in its physically stable helical state is disrupted. This way, different types of proteins can couple to a DNA or RNA strand and access its information. Examples of such proteins are topoisomerases, helicases and polymerases.

In step a) of the method according to the invention a first end of the macromolecule is provided with a paramagnetic marker, typically by chemical bonding. An example of a useful non-covalent bond between the macromolecule and the paramagnetic marker is a protein-ligand bond, such as a biotin-streptavidin bond.

The paramagnetic marker is attached directly to the macromolecule. In the context of the present invention, directly attaching the paramagnetic marker to the macromolecule also comprises the use of an intermediate molecular moiety to enable and/or enhance the attachment of the paramagnetic marker to the macromolecule. Such an intermediate molecular moiety then functions as molecular glue, in analogy with mortar that is used to inseparately attach two bricks. It is noted that said intermediate molecular moiety only serves the purpose of specific attachment. The intermediate molecular moiety has small dimensions compared to the dimensions of the paramagnetic marker and the macromolecule, i.e. the intermediate molecular moiety should have typical dimensions that are about 10 times or more smaller than the dimensions of the paramagnetic marker. Typically, the intermediate molecular should have dimensions between 5 and 500 nm, preferably between 100 and 250 nm. For instance, when attaching a paramagnetic marker to a DNA construct, advantageously the DNA construct is ligated at the respective end to a functionalized DNA PCR fragment having e.g. multiple biotin groups. The paramagnetic marker is then functionalized with streptavidin or neutravidin. Other protein-ligand bonds that can be used are digoxigenin-antidigoxigenin bonds or fluorescein-antifluorescein bonds.

It is noted that the use of an intermediate molecular moiety to attach the paramagnetic marker to the macromolecule is different from the situation in Celedon et al., who use a nanorod-assembly to attach a macromolecule to the paramagnetic marker. This nanorod-assembly, a 2 µm Ni-Pt nanorod attached to a 0,1 µm Ni-segment, is indeed a functional mechanical part of the nanorod-paramagnetic marker-macromolecule-assembly. The nanorod is needed to enable and detect rotation of the macromolecule. Without the nanorod, no torque can be measured. This is different from the intermediate macromolecular moiety, which plays no role in the determination of the position or orientation of the paramagnetic marker. If chemically possible, the intermediate macromolecular moiety could have been omitted.

Apart from that, the nanorod according to Celedon et al itself is attached to the macromolecule by means of the above intermediate macromolecular moieties, i.e. by means of neutravidin-biotin/digoxigenin bonds.

Finally, the nanorod according to Celedon et al has a length of about 2 µm, which is much larger than the intermediate molecular moieties according to the present invention.

Thus, in the context of the present application the nanorod is not analogous to an intermediate molecular moiety.

Preferably, the protein-ligand bond used to tether the macromolecule to the surface of the holding means does not interfere with the protein-ligand bond used to mount the macromolecule to the paramagnetic marker.

The paramagnetic marker can be a spherical particle, but the paramagnetic marker may also have another shape, such as cubical, or irregular. A spherical particle like a bead has the advantage that the calibration procedures to be followed when implementing a measurement can be simplified by assuming that the particles are more or less spherical.

The paramagnetic character of the paramagnetic marker may be an intrinsic property of the material of which the paramagnetic marker is made. Advantageously, the paramagnetic marker comprises a polymer matrix comprising e.g. embedded ferro paramagnetic particulates such as molecules or grains. A non-magnetic carrier coated with a paramagnetic coating may also be used. Preferably, the polymer is polystyrene. Streptavidin coated Dynabeads ® (Invitrogen) are an example.

Advantageously, the paramagnetic marker of the macromolecule-paramagnetic marker-assembly comprises visualizing elements to facilitate visualization of the rotations of the macromolecule-paramagnetic marker-assembly. Visualization can be imparted by fluorescent compounds, for example by embedding the paramagnetic marker with fluorescent molecules and /or grains.

The macromolecule-paramagnetic marker-assembly can be prepared easily using standard, i.e. commercially available, techniques and materials. Furthermore, the preparation of the macromolecule-paramagnetic marker-assembly can be done within minutes time, without the need to synthesize a nanorod to allow for angular tracking of the paramagnetic marker. According to the invention the paramagnetic marker is attached to the macromolecule without the insert of an asymmetry inducing connection. Another advantage of the method according to the invention is that it allows for a high density of suitable assemblies per unit of surface area. Using the method according to the invention, about 1 molecule per 40 square microns can be measured. When imaging the assemblies with a 100x objective with a 60x44 µm² field of view of, this means that about 60 assemblies can be measured simultaneously. It will be recognized that the method according to the invention is not limited to the use of the coupling moieties functionalizing the paramagnetic marker, but that also other ligand-protein bond forming moieties may be used.

The macromolecule provided with the paramagnetic marker forms a macromolecule-paramagnetic marker-assembly. In step b) of claim 1, this assembly is tethered to one or more tethering points of a holding means, e.g. a glass plate such as a microscope cover slip. Generally this tethering is done by chemically attaching the end of the macromolecule which is not provided with the paramagnetic marker to said one or more tethering points. Preferably, the assembly is tethered to more than one tethering point, since tethering the assembly to one tethering point allows the assembly to rotate more or less freely around the tether. Again, the attachment may be enabled and/or enhanced by suitable modification of the surface of the holding means and/or of the respective end of the macromolecule, such as a digoxigenin functionalized DNA PCR fragment or a digoxigenin functionalized RNA transcript, in either case containing multiple digoxigenin labels. In order to tether the macromolecule-paramagnetic marker-assembly to the tethering point of the holding means, preferably the surface of the holding means comprising the tethering point is functionalized as well, e.g. with anti-digoxigenin groups.

The tethering point is situated in an x,y-plane such that the macromolecule-paramagnetic marker-assembly is arranged substantially in the z-direction perpendicular to the x,y-plane, between the main magnet arranged substantially perpendicular to the x,y-plane and the surface of the holding means. The magnetic field generated by the main magnet is directed substantially in the z-direction at the position of the macromolecule-paramagnetic marker-assembly. Advantageously, the main magnet is disposed above the holding means such that the z-direction coincides with the vertical direction. The pulling force exerted on the paramagnetic marker of the macromolecule-paramagnetic marker-assembly depends on the distance in the z-direction between the main magnet and the paramagnetic marker, i.e. the smaller the distance, the higher the pulling force exerted on the paramagnetic marker. Thus, the more accurately the distance between the main magnet and the paramagnetic marker can be controlled, the more accurately the applied force can be controlled. In the method according to the invention the distance between the main magnet and the paramagnetic marker can be established with an accuracy of about 1 µm. In step c) of the method according to the invention, a starting position of the paramagnetic marker of the macromolecule-paramagnetic marker-assembly is determined in a magnetic field generated by the main magnet. This starting position is not a fixed position, but a time-averaged position. The position of the paramagnetic marker in the magnetic field fluctuates due to thermal fluctuations. In the starting position, the macromolecule may be in its torsionally relaxed state. Another possibility is to prewind the macromolecule to a starting position and then carry out the method according to the invention.

In step d) of the method according to the invention, the main magnet is rotated a predetermined number of times around its predominant magnetic axis. This causes the paramagnetic marker to rotate around the magnetic axis of the main magnet as well. The magnet is rotated a predetermined number of times N. In this context, N may be a real number. Preferably, N is an integer.

After N rotations, the paramagnetic marker of the macromolecule-paramagnetic marker-assembly adopts a measuring position. Again this measuring position is a time-averaged position because of the thermal fluctuations. In this measuring position, the macromolecule-paramagnetic particle marker-assembly exerts a restoring torque. As a consequence of this torque, generally there is a difference between the starting position and the measuring position, i.e. between the position in the x,y-plane and the angular orientation in the x,y-plane of the starting position and the measuring position. Said difference is measurable due to the low torsional stiffness of the angular trap induced by the magnetic field.

If the characterizing feature is torque the difference in position and the angular deviation of the paramagnetic marker need to be determined. This may be done by making use of small irregularities in the shape of the paramagnetic marker, which provide an asymmetry to the macromolecule-paramagnetic marker-assembly and hence in the paramagnetic marker image. Another option is to use an angular tracking marker, which also provides an asymmetry in the paramagnetic marker image. The irregularities and the angular tracking marker allow for the determination of the angular deviation in the position of the paramagnetic marker in the x,y-plane in the measuring position with respect to the starting position in step e).

The angular tracking marker is preferably non-magnetic and has the function of enabling the determination of the angular deviation of a measuring position of the paramagnetic marker with respect to a starting position in step e). Without the angular tracking marker, the rotations of the paramagnetic marker, induced by the applied magnetic field, would be difficult to track.

Like the paramagnetic marker, advantageously the angular tracking marker is a particulate, preferably a spherical particle having a smaller size than the paramagnetic marker. This way, the angular tracking marker does not significantly perturb the spherical shape of the paramagnetic marker. This is advantageous since tracking and calibration procedures to calibrate pulling procedures assume an overall spherical shape of the macromolecule-paramagnetic marker-assembly. Thus, the more spherical the overall shape of the assembly, the more accurate the calibration procedures.

The angular tracking marker may comprise a polymer matrix comprising optionally fluorescent particles, such as fluorescent compounds and/or grains. Such a marker is exemplified by Fluorospheres® microspheres (Invitrogen). These microspheres can be attached to the paramagnetic marker using standard bioanalytical techniques. In order to attach the angular tracking marker to the paramagnetic marker, said angular tracking marker is functionalized with biotin, which couples to a streptavidin functionalized paramagnetic marker. Typically, when attaching angular tracking markers to the paramagnetic markers, one or more angular tracking marker attaches to the paramagnetic marker. In practice, the accuracy of the method is not significantly influenced by the number of angular tracking markers. Preferably, each paramagnetic marker has one angular tracking marker attached.

Optionally, also the paramagnetic marker is embedded with fluorescent molecules and/or grains. Advantageously, the rotation angle of the paramagnetic marker is preferably tracked via cross-correlation analysis of CCD images of the paramagnetic marker.

From the determined angular deviation of the paramagnetic marker one or more characterizing features of a macromolecule can be calculated. Examples of such characterizing features are torque, twist, torsional stiffness, buckling torque, and critical torques such as those governing phase transitions of the macromolecule or the unbinding of protein moieties from the macromolecule. It is noted that in the present context the changes in twist induced in a macromolecule are measured and not the absolute value of the twist of a macromolecule. For convenience reasons however, the 'changes in twist' are referred to as 'twist'.

In a preferred embodiment of the invention, the torque of a macromolecule is determined, i.e. the restoring torque a macromolecule exerts in response to a predetermined number of rotations of the paramagnetic marker.

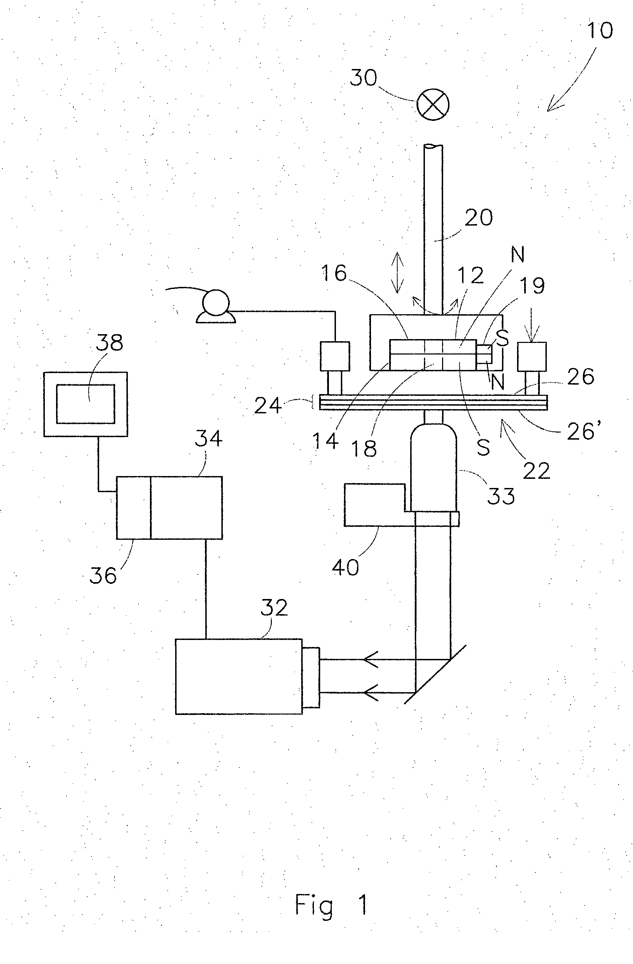

Surprisingly, the inventors were able to apply said forces and torques without the need of a nanorod as described by Celedon et al. In said article the authors claim that the torque is dependent on the horizontal component L_{H} of the distance between the attachment point of the probe to the macromolecule and the magnetic centre of the probe (page 1721, "Controlled Introduction of Turns into the Molecule"; caption of figure 1). It follows that in order to be able to apply a torque to the macromolecule, L_{H} should be non-zero. However, when applying torque to a macromolecule with a configuration according to the present invention, the macromolecule may be attached to the south pole of the paramagnetic marker, meaning that there is no horizontal component L_{H}, and, following Celedon et al, meaning that it should be impossible to apply a torque to the macromolecule. Yet, the inventors were able to apply a torque to the macromolecule, due to the use of a side-magnet in combination with the main magnet. The auxiliary magnetic field exerted by the side-magnet causes an asymmetry in the magnetic field generated by the main magnet, the asymmetry causing the paramagnetic marker to rotate when the main magnet having the side magnet attached thereto is rotated. That the present inventors were able to apply torque, even with a macromolecule-paramagnetic marker-assembly with a zero horizontal component L_{H}, is a strong indication that the present invention involves an inventive step.

To enable the determination of said torque, an auxiliary magnetic field is generated by means of a side magnet arranged adjacent to the main magnet. For practical reasons, the magnetic axes are preferably arranged in parallel. This auxiliary magnetic field provides a horizontal component to the magnetic field of the main magnet, provding a sufficient asymmetry to the predominently vertically oriented field to allow for rotation of the paramagnetic marker. The poles of the side magnet may be arranged in such a way that either the poles of the side magnet are parallel or the poles are opposite to the poles of the main magnet. Advantageously, the poles of the side magnet are arranged opposite to the poles of the main magnet.

Said side magnet may be a permanent magnet, but can also be an electromagnet.

Preferably, the strength ratio of the magnetic force in the predominant pulling direction, i.e. the z-direction) exerted by the main magnet compared to the force exerted by the side magnet is within the range of 10,000:1 to 10:1. In this range the addition of the side magnet requires no recalibration of the overall magnetic force. Additionally, the strength ratio of the magnetic force in the predominant pulling direction (z) exerted by the main magnet and the magnetic force in the transverse direction (x or y) exerted by the side magnet is within the range of 10,000:1 to 10:1.

Advantageously, the ratio of torques to be measured in the macromolecule and the torsional stiffness of the trap *kᵣₒₜ* are such that the difference between the angular orientation of the measuring position and the angular orientation of the starting position is in the range of 0.1 degrees to 100 degrees, preferably between 0.1 degrees and 10 degrees.

Advantageously, the side magnet consists of an assembly of electromagnetic coils comprising two spaced apart x-coils that extend in the horizontal x-direction and two spaced apart y-coils that extend in the horizontal y-direction, wherein the electromagnetic coils are powered by current sources through electrical wires. In this configuration the electromagnetic coils are positioned in such a way that the holding means is embedded in the space enclosed by said electromagnetic coils.

In yet another preferred embodiment of the invention, in step b) of claim 1 the tethering point is aligned with the magnetic axis of the main magnet. The side magnet is absent, as a result of which the stiffness of the angular trap *kᵣₒₜ* is reduced to a level below that of typical macromolecules.

Surprisingly, the inventors found that when the main magnet and the tethering point of the holding means are relatively positioned such that the magnetic axis of said main magnet and the tethering point accurately coincide, the paramagnetic marker circulates around the tethering point, thereby following a circular path around the tethering point. Said coinciding is defined with respect to the tethering of the macromolecule to the surface. This circulating takes place without rotation of the external magnetic field, , since it is driven by the thermal fluctuations that cause change in the twist of the macromolecule and a resulting response of the paramagnetic marker. More precisely, in this specific measuring position at nearly zero field in the x,y-plane, the circular motion of the paramagnetic marker coincides with a rotation about its tethering point, as verified by employing the angular tracking marker. This simultaneous rotation and circular motion is analogous to the motion of the moon around the Earth, in which the moon rotates about its own axis at the same rate as its rate of circulation about the Earth (as a result of which an Earth-based observer always views the same side of the moon).

It follows that monitoring the position of the paramagnetic marker on the circular path during motion is an alternative way to monitor angular fluctuations or excursions of the macromolecule, without the need for an angular tracking marker. Said circular path has a radius on the micrometer scale, sufficient to be adequately measured. The circular motion may thus be employed to determine changes in twist of the macromolecule. The conversion of x,y-coordinates along a circular path or arc to angular coordinates can also be used to measure the torque in a macromolecule. It is noted that in the latter case rotation of the main magnet and an auxiliary magnetic field exerted by a side magnet, as described above, are used to apply said torque

Said magnetic axis of the main magnet and said tethering point coincide within a margin of approximately 10 preferably 1, more preferably 0.1 µm,.

It turns out that in this embodiment the radius of the circular path has a maximum value on the order of the radius of the paramagnetic marker and a minimum value of zero. The exact value depends on the point on the surface of the paramagnetic marker where the macromolecule is attached to the paramagnetic marker.

In a preferred embodiment of the methods according to the invention steps c) and e) comprise illumination of the macromolecule-paramagnetic marker-assembly with an illumination means emitting radiation, measuring the transmission, scattering, or reflection of the emitted radiation on the paramagnetic marker and/or the angular tracking marker, and imaging the data related to radiation's interaction with the paramagnetic marker and/or the angular tracking marker.

In another preferred embodiment the macromolecule-paramagnetic marker-assembly is illuminated by the illumination means through a main magnet that is hollow. Hollow in this context means that a passage from one side to the other side of the main magnet is present along the optical axis of said illumination.

Advantageously the pulling force applied by the main magnet on the macromolecule is in the range of from at least 0.001 to 1000, preferably 0.01 to 100, more preferably 0.1 to 10 pN. Very high pulling forces above 500 pN are useful to measure stiffer molecules such as actin filaments or microtubules.

The invention also relates to an apparatus for performing the methods described above. The apparatus comprises:
- holding means for holding the macromolecule, said holding means comprising one or more tethering points to which at least one end of the macromolecule is tethered, said tethering point being situated in a x,y-plane such that the macromolecule-paramagnetic marker-assembly is arranged substantially in the z direction perpendicular to the x,y-plane, between a main magnet arranged substantially perpendicular to the x,y-plane and the surface of the holding means;
- a main magnet for generating a magnetic field for applying a force to the macromolecule, said main magnet being rotatable around its magnetic axis and arranged at a controllable distance from the holding means; the magnetic axis being arranged perpendicular to x,y-plane of the holding means, wherein the poles of the main magnet are arranged in the z-direction and wherein the main magnet generates a magnetic field directed substantially in the z direction at the position of the macro-molecule-paramagnetic marker-assembly;
- means for rotating the main magnet; and
- imaging means for imaging information related to the position of the paramagnetic marker and/or angular tracking marker.

The main magnet according to the invention may be a permanent magnet, but also an electromagnet. As explained above, the force the paramagnetic marker experiences can be varied by varying the distance in the z-direction between the main magnet and the holding means. An electromagnet has the additional advantage that the force can also be varied by varying the amount of current that goes through the main magnet.

According to the invention, the apparatus comprises rotating means for rotating the magnet around its magnetic axis. Advantageously, said rotating means also comprise translating means for translating the main magnet in the z-direction.
- imaging means for imaging information related to the position of the paramagnetic marker and/or angular tracking marker, which apparatus further comprises a side magnet for allowing the macromolecule provided with a paramagnetic marker to rotate when rotating the main magnet.

The main magnet according to the invention may be a permanent magnet, but also an electromagnet. As explained above, the force the paramagnetic marker experiences can be varied by varying the distance in the z-direction between the main magnet and the holding means. An electromagnet has the additional advantage that the force can also be varied by varying the amount of current that goes through the main magnet.

According to the invention, the apparatus comprises rotating means for rotating the magnet around its magnetic axis. Advantageously, said rotating means also comprise translating means for translating the main magnet in the z-direction.

The side magnet is attached to a side of the main magnet.

Said side magnet may be a permanent magnet that is independently translatable from the main magnet, or said side magnet may be an electromagnet. In both of these advantageous scenarios, the strength of the torque clamp and the pulling force can be varied separately.

In an advantageous embodiment of the present invention the side magnet consists of an assembly of electromagnets, i.e. Helmholtz coils, comprising two spaced apart x-coils that extend in the horizontal x-direction and two spaced apart y-coils that extend in the horizontal y-direction, wherein the electromagnetic coils are powered by current sources through electrical wires, wherein the electromagnetic coils are positioned in such a way that the holding means is embedded in the space between said electromagnetic coils.

By using a combination of electromagnets and permanent magnets, the torque applied to the paramagnetic marker can be uncoupled from the pulling force, which introduces an additional degree of freedom in controlling the paramagnetic marker.

In an apparatus for measuring twist, the magnetic axis of the main magnet and the tethering point of the holding means can be relatively positioned such that the magnetic axis of said main magnet and the tethering point coincide, within a margin of approximately 10, preferably 1, more preferably 0,1 µm.

The preferred embodiments described with respect to the method according to the invention are similarly applicable to the apparatus.

Advantageously the apparatus further comprises a light source for illuminating the macromolecule-paramagnetic marker-assembly, wherein the light source is positioned in the z-direction such that the main magnet is between said light source and the macromolecule-paramagnetic marker-assembly, and wherein the main magnet is hollow to allow radiation surface of the holding means to which the macromolecule is tethered, such that the macromolecule is inside the inner chamber.

Generally, the imaging means comprises optical magnification means for visualizing the position of the paramagnetic marker and/or angular tracking marker, detector means for detecting said position of said paramagnetic marker and display means for displaying said information. In a particular embodiment the detector is a CCD camara. The images provided with said CCD camera are submitted to an image analysis routine with a subpixel fitting step. This way the position of the paramagnetic marker in the x,y-plane can be determined with a resolution of about 1 nm and about 0.1 degree. Typically the apparatus also comprises recording means for recording the position of the marker and/or the calculated characterizing features.

In a particularly preferred embodiment the holding means comprises one or more reference elements attached to at least one surface of the holding means to which the macromolecule is tethered, as a reference point for the position of the paramagnetic marker.

The various aspects of the invention are further illustrated by means of the attached drawings, wherein:
Fig. 1 shows a diagrammatically represented embodiment of a device for measuring torque in a macromolecule according to the invention;
Fig. 2 shows the position of a torsionally relaxed macromolecule;
Fig. 3 shows the position of a macromolecule after rotating N turns;
Fig. 4 is an image of the position of the paramagnetic marker with the attached angular tracking marker, this assembly being attached to a macromolecule; the image is recorded with the focussing plane being about 5 µm above the focal plane of the paramagnetic marker;
Fig. 5 is a comparison between the traces of rotational fluctuations obtained from analysis of CCD images for a surface immobilized bead (upper signal), for a DNA tethered bead held in conventional magnetic tweezers (see e.g. US2003/0166262 A1) (middle signal) and for a DNA tethered bead according to the present invention;
Fig. 6-9 show the torque results for dsDNA.
Fig. 10 shows an embodiment for measuring torque with electromagnetic coils as a side-magnet;
Fig. 11 shows a plot of the (x,y)-fluctuations as a function of increasing magnetic field exerted by the electromagnetic coils;
Fig. 12 is a diagrammatically represented embodiment of a device for measuring twist in a macromolecule according to the invention;
Fig. 13 shows the position of the tethered macromolecule and a detail of the paramagnetic marker tethered to a macromolecule.
Fig. 14 shows a plot of the (x,y)-fluctuations measured with the embodiment of fig.10 showing thermal fluctuations of the paramagnetic marker.
Fig. 15 shows a comparison of the rotation angle determined from direct tracking of a angular tracking marker and from the paramagnetic marker's position in the (x, y)-plane (a) and a plot of the turns the paramagnetic marker makes as a function of time (b, c); fig. 15b is obtained by converting the X-Y-plot of fig. 14 to an angle.
Fig. 16 -18 show the results of monitoring the assembly of recombination protein A (RecA) filaments on DNA.

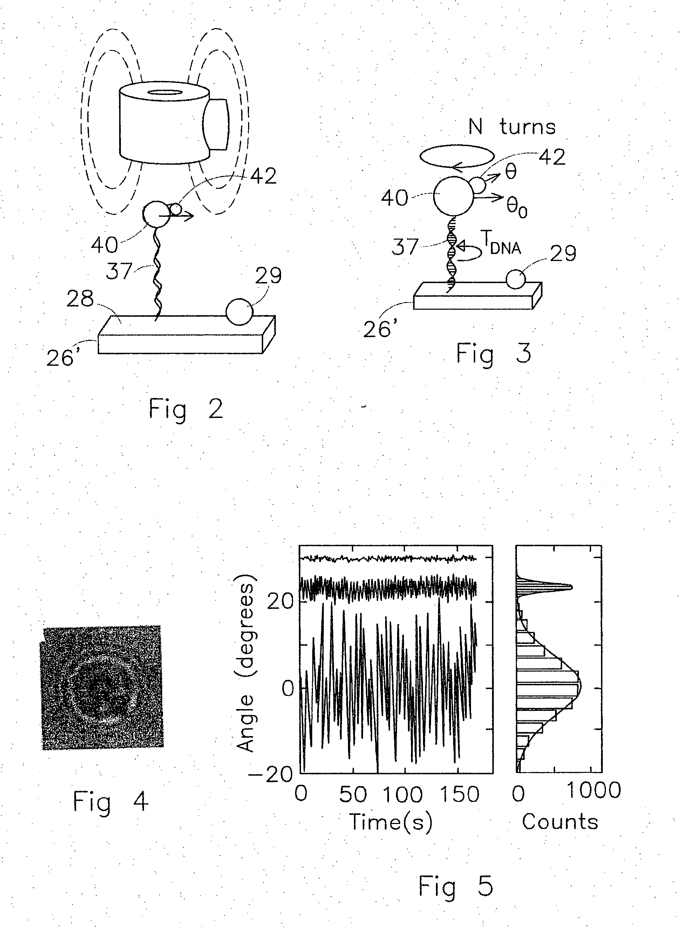

In Fig. 1 a device 10 for measuring a characterizing feature of a macromolecule is shown diagrammatically. The device 10 comprises a vertically arranged main magnet 12 designed to apply force while reducing kᵣₒₜ having its south pole 14 at the bottom and its north pole 16 at the top. The main magnet 12 defines a centre hole 18 for illumination purposes as will be explained later. A smaller side magnet 19 for applying torque having its poles opposite to those of the main magnet 12 is mounted to the cylindrical outer wall of the main magnet 12 allowing bead rotation of the macromolecule to be tested. The resulting magnetic field is represented with dashed lines, see Fig. 2. The magnets 12 and 19 are mounted in a frame 20, which can be rotated and translated in vertical direction by means of motors (not shown). Below the main magnet 12 a holding means 22 is arranged, that can be translated on nanometer scale in the x,y-plane. In the illustrated embodiment the holding means 22 comprises a flow cell 24 made from parallel arranged transparent microscope cover slides 26, e.g. made from glass, spaced apart a small distance using watertight spacers. The inner surface 28 (see Fig. 2 and 3) of the bottom slide 26' is coated with sticky agents such as nitrocellulose or polystyrene. To the now sticky inner surface 28 of the bottom slide 26, one or more non-magnetic references 29 for correction of mechanical drift- here non-magnetic latex beads having a diameter of for example about 3.0 micrometers - are non-specifically attached. An illumination means 30, e.g. one or more LEDs and a focussing lens are mounted in the frame and aligned with the centre hole 18. Below the flow cell 24 an imaging means 32 such as a CCD camera and/or photodetector and suitable magnification means 33 such as a 100x oil immersion objective is positioned and also aligned with the illumination means 30 and centre hole 18. Below the magnification means 33 a piezo-controlled nanometer stage 40 is positioned, which allows for the precise adjustment of the focal plane of the objective in the z-direction. The imaging means 32 are connected to a computer 34 for receiving signals from the imaging means 32, analyzing the signals and calculating the characterizing feature(s). Usually the signals and data will be recorded in a memory 36 of the computer 34. The computer 34 is provided with a screen 38 for displaying the information received and features calculated and a printer (not shown).

The reference beads 29 can be attached at random to the slide 26' by incubation in phosphate buffered saline (PBS buffer) for about half an hour.

A macromolecule 37 is provided with a paramagnetic marker 40 and tethered to the holding means 22. For example a DNA construct is ligated at both ends to functionalized DNA PCR fragments, in particular a first PCR fragment having multiple biotin groups and a second PCR fragment having multiple digoxigenin groups. In order to attach the macromolecule to the holding means 18 the surface is functionalized by non-specifically attaching anti-digoxigenin moieties to the inner surface of the lower cover slide 26, to which the digoxigenin functionalized PCR fragment of the macromolecule binds. After this, the inner surface 26' is passivated by contacting said surface with blocking agents such BSA (bovine serum albumin), poly-L-glutamic acid or casein.

The biotin PCR fragment tethers to the paramagnetic marker, e.g. streptavidin-coated superparamagnetic beads M270 having a 2.8 micrometer diameter, available from Invitrogen). After tethering, an angular tracking marker, e.g. one or more smaller non-magnetic beads 42, such as biotin-labelled Fluosphere microspheres (also available from Invitrogen) are attached to the larger paramagnetic marker 40 of the marker-macromolecule assembly by incubation of the flow cell during a sufficient period of time with a predetermined diluted solution of these microspheres. Typically one or two or even more microspheres are attached to a magnetic bead. These fluorescent microspheres provide visual asymmetry to the assembly sufficient to provide similar asymmetry of the bead image to track rotation, see fig. 4.

More specifically the smaller angular tracking bead allows the tracking of the position and the rotation angle via cross-relation analysis of defocused CCD images with an accuracy of about 0.1° using the following algorithm. A defocused CCD image of a paramagnetic bead and smaller microsphere showing the asymmetry is obtained. The symmetry center of the paramagnetic bead image is determined by a position tracking algorithm. Around this symmetry center, a radial section comprising the asymmetry is selected and polar transformation is carried out. Radially averaging the defocused image and its own mirror image provides two angular signatures. The cross-correlation of these angular signatures offers a maximum indicating the symmetry shift. Sub-pixel fitting to determine angular position using a selection around the maximum previously determined as an angular fit yielding an angular position at sub-pixel resolution.

Using the above configuration, the macromolecule thus tethered is imaged first in its torsionally relaxed state (=0 turns) as shown in Fig. 2. From analysis of the CCD images, the bead positions in the x, y and z direction are determined (Science 271: 1835-1837). Then the rotationally arranged magnets 12 and 19 are rotated for a predetermined number of times N in a given direction, e.g. in case of a helically configured double strands DNA overwinding (i.e. in the direction of the helix) or underwinding (in opposite direction). See Fig. 3. Then again the paramagnetic marker connected to the torsionally tensioned macromolecule is imaged in a slightly defocused way. See Fig. 4.

Fig. 5 compares the traces of rotational fluctuations obtained from analysis of CCD images for a surface attached bead (upper signal and histogram), for a DNA tethered bead held in conventional magnetic tweezers (see e.g. US2003/0166262 A1) (middle signal and histogram) and for a DNA tethered bead according to the present invention (middle signal and histogram). The stretching force is 3 pN for all traces.

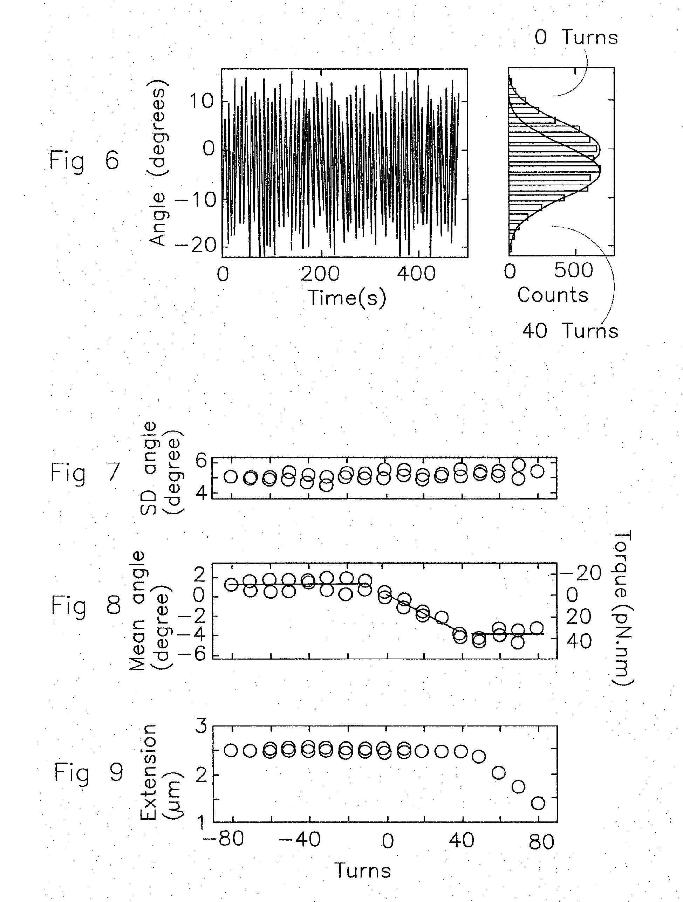

Fig. 6-9 show torque measurements for dsDNA, in particular a 8 kilobasepairs (kbp) DNA molecule at a stretching force of 3.5 pN in PBS buffer. Fig. 6 shows the Gaussian distribution of angular fluctuations of DNA tethered beads for torsionally relaxed DNA (starting position = 0 turns) and for N=40 turns. The shift of the distribution centre due to the restoring torque exerted by DNA is apparent. Fig. 7 shows that the width of the angular distributions and therefore the trap stiffness are constant for the range of torques exerted by DNA. Fig. 8 shows the mean angle (left Y axis) as function of the number of turns, which can be directly converted to torque (right Y axis) using the equations *kᵣₒₜ = k_{B}T*/(δθ²) and *T_{Macromolecule} = kᵣₒₜ*(θ- θ₀). The buckling torque can be determined from the plateau in the torque vs. turn data at positive turns in Fig. 8. The twist persistence length C of DNA as a function of force can be determined from linear fits of these data in the elastic twist regime. Fig. 9 shows the extension of the tethered DNA.

Using the apparatus the macromolecule tether extension and torque response are determined while over- and underwinding single macromolecules. Starting with a torsionally relaxed molecule at 0 turns, DNA initially behaves like an isotropic flexible rod: as positive turns are added to the molecule, its extension remains approximately constant and the shift in the mean angular signal decreases linearly with increasing number of turns. Since in the range of small angular excursions the stiffness of the torsional trap is independent of the applied number of turns, this indicates that torsional strain builds up linearly in the molecule. Since the torque after *N* turns *T(N)* can be equated to *2πNk_{B}TC*/*L_{c},* where C and *L_{c}* are the twist persistence length and contour length of DNA, respectively, C is determined directly from the linear slope of the torque response (*L* is determined from independent DNA stretching experiments). As more positive turns are added, the build up in torsional strain continues until a critical twist density is reached where the molecules buckles to form plectonemic supercoils. Beyond this transition the torque in the molecule remains constant at *Tb* (buckling torque) and additional turns lead to an accumulation of plectonemic supercoils associated with a rapid decrease in extension. When negative turns are applied to torsionally relaxed DNA, the DNA likewise initially behaves like an isotropic rod and exerts a restoring torque with opposite sign compared to the situation at positive turns. Under tension of 1 pN or greater, DNA does not buckle when the underwinding (the extension of the molecule remains approximately constant, and instead the molecule denatures locally. The corresponding denaturation torque *T_{denat} =* -10 ± 1 pN.nm.

Systematic torque response measurements on single DNAs were performed to determine the buckling torque *Tb* and twist persistence length C as a function of the stretching force *F. Tb* increases systematically with_increasing *F*. A simple model of DNA elasticity predicts *Tb =* (*2k_{B}TLₚF*)^{*1*/}*²,* where *Lₚ* is the bending persistence length that was determined from independent stretching experiments.

The effective torsional stiffness of DNA C that was derived from linear fits of the torque as a function of turns exhibits a significant F-dependence:

The direct measurement of torque using the method and apparatus according to the invention has the advantage that it can also probe the torsional properties of complex structures, such as DNA-protein filaments. The RecA heteroduplex was examined, which in living cells is a product of strand invasion of duplex DNA by RecA-coated single-stranded DNA. Filaments of the recombinase RecA play a central role in the homologous recombination DNA repair pathway.

RecA was assembled in the presence *of* ATP-γS on torsionally constrained DNA, employing magnet rotation to fully assemble the RecA filament in its torsionally relaxed state. The relaxed heteroduplex was shifted by -330 turns with respect to relaxed bare, as expected for full RecA coverage of the 7.9 kbp DNA construct given the observed unwinding by 15° per bp upon RecA binding. Next over- or underwinding the RecA heteroduplex was performed while measuring both extension and torque. The initial build up of torque can be fitted to yield *C_{RecA},* which was found to be 1.8-fold larger than that of bare DNA at the same force.

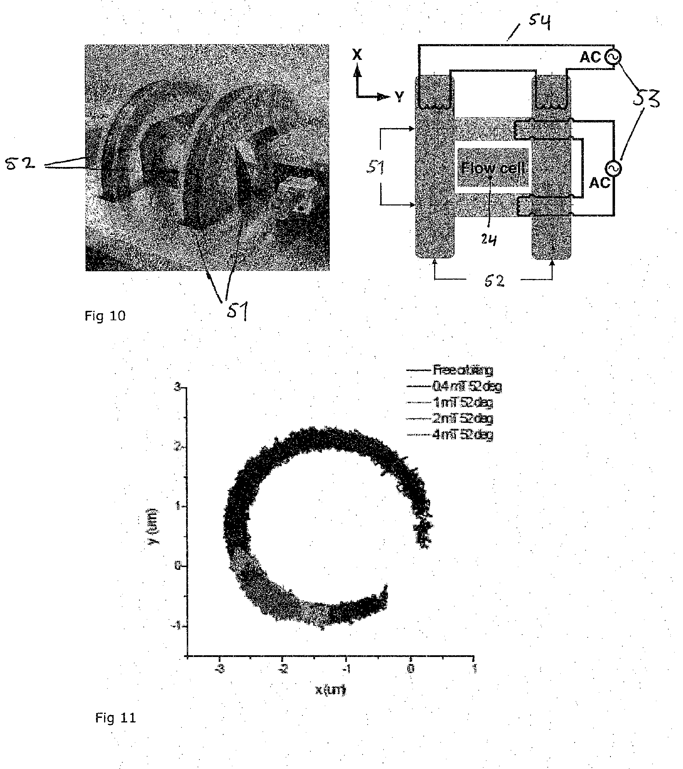

Fig. 10 shows an alternative embodiment of the present invention. A device for measuring the torque in a macromolecule is equipped with an assembly of electromagnetic coils 51, 52 as a side magnet 19. The left picture shows a side view of such an assembly, whereas the right picture depicts a schematic top view of the assembly. The assembly consists of two spaced apart x-coils 51 and two spaced apart y-coils 52. In the particular embodiment, the distance between the two y-coils is about 10 cm and the distance between the x-coils is about 5 cm. It is noted that these dimension can be adjusted at will and by no means limit the scope of the invention. As can be seen on the right picture, the electromagnetic coils 51, 52 are powered by any suitable power supply, e.g. an alternating current supply, through electrical wires 54.

The electromagnetic coils 51, 52 are positioned in such a way that the flow cell 22 is embedded in the space between the said electromagnetic coils 51, 52 .

By using electromagnetic coils 51, 52 as a side magnet 19 a homogeneous field can be created merely in the plane parallel to the flow cell 22. This field that is used to apply the torque can be controlled independently from the upward pulling force exerted by the main magnet 12. By using the combination of electromagnetic coils 51, 52 and a permanent magnet, the torque applied to the paramagnetic marker 40 can be uncoupled from the pulling force of the main magnet 12 , which introduces an additional degree of freedom in controlling the paramagnetic marker 40 .

Fig.11 shows some plots of the fluctuations of a paramagnetic marker 40 under increasing magnetic field strength of the electromagnetic coils 51, 52 under a constant pulling force of the main magnet 12. When the electromagnetic coils are off, the angular fluctuations lie on an almost circular path. Upon switching on the electromagnetic coils 51, 52 the angular fluctuations are more and more constrained upon increasing the magnetic field exerted by the electromagnetic coils 51, 52 . The advantage of using electromagnetic coils 51, 52 is that the field exerted by these coils can be adjusted by simply adjusting the current delivered to the coils, i.e. turning a knob. When trying to obtain the same plot with a permanent side-magnet 19 , it would be necessary to adjust the distance between the side-magnet and the paramagnetic marker 40 , in a reproducible way.

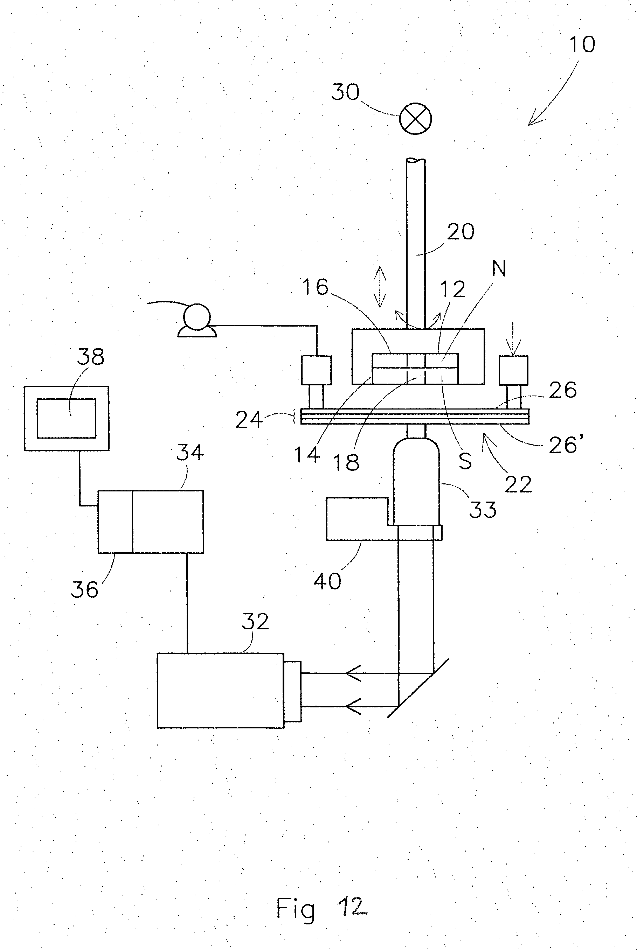

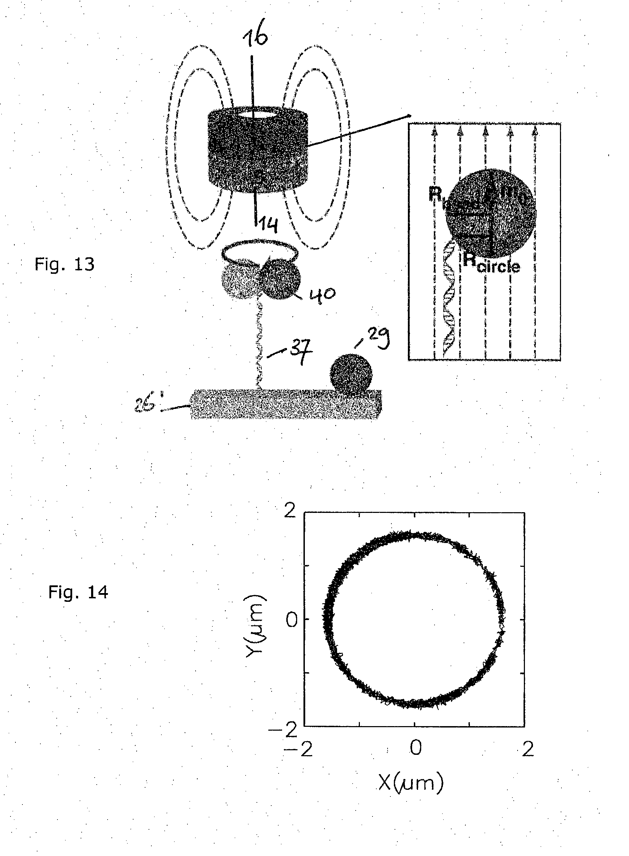

Fig. 12 is a diagram showing an embodiment of a method and apparatus for measuring twist in a macromolecule. Parts similar to those in Fig. 1-3 are indicated by the same references. The device 10 for measuring twist of a macromolecule comprises a vertically arranged main annular magnet 12 having its south pole 14 at the bottom and its north pole 16 at the top. Thus the main magnet 12 defines a centre hole 18. Contrary to the torque measuring device as shown previously this embodiment lacks the smaller side magnet 19. The resulting magnetic field is represented with dashed lines in the left part of Fig.13. The main magnet 12 is mounted in a frame 20, which can be rotated and translated in vertical direction by means of a motor (not shown). Below the main magnet 12 a holding means 22 is arranged, that can be translated on nanometer scale in the x,y-plane. In the illustrated embodiment the holding means 22 comprises a flow cell 24 made from parallel arranged transparent microscope cover slides 26, e.g. made from glass, spaced apart a small distance using spacers. The inner surface 28 (see Fig. 2 and 3) of the bottom slide 26' is coated with nitrocellulose. One or more non-magnetic references 29 for correction of mechanical drift-here non-magnetic latex beads having a diameter of for example about 3.0 micrometers - are attached to the inner surface 28 of the bottom slide 26'. An illumination means 30, e.g. one or more LEDs, are mounted in the frame and aligned with the centre hole 18. Below the flow cell 24 an imaging means 32 such as a CCD camera and suitable magnification means 33 such as a 100x oil immersion objective is positioned and also aligned with the illumination means 30 and centre hole 18. Below the magnification means 33 a piezo-controlled nanometer stage 40 is positioned, which allows for the precise adjustment of the focal plane of the objective in the z-direction. The imaging means 32 are connected to a computer 34 for receiving signals from the imaging means 32, analyzing the signals and calculating the characterizing feature(s). Usually the signals and data will be recorded in a memory 36 of the computer 34. The computer 34 is provided with a screen 38 for displaying the information received and features calculated and a printer (not shown).

The reference beads 29 can be attached at random to the slide 26' by incubation in PBS buffer for about half an hour.

A macromolecule 37 is provided with a paramagnetic marker 40 and tethered to the holding means 22. For example a DNA construct is ligated at both ends to functionalized DNA PCR fragments, in particular a first PCR fragment having multiple biotin groups and a second PCR fragment having a plurality of digoxigenin groups. In order to attach the macromolecule to the holding means 22 the surface is prepared by functionalizing the inner surface of the lower cover slide 26' with anti-digoxigenin, to which the digoxigenin functionalized PCR fragment of the macromolecule binds. The biotin PCR fragment tethers to the paramagnetic marker, e.g. streptavidin-coated superparamagnetic beads M270 having a 2.8 micrometer diameter, available from Invitrogen).

In order to determine twist, the tethered molecule is arranged with its main axis coinciding with the longitudinal axis of the magnet 12. This way the paramagnetic marker circulates around the tethering point, thereby following a circular path around the tethering point. This circulating is driven by the thermal fluctuations that cause change in the twist of the macromolecule and a resulting response of the paramagnetic marker. In this specific measuring position at nearly zero field in the x,y-plane, the circular motion of the paramagnetic marker coincides with a rotation about its tethering point, as explained above. See the right part of fig. 13. The predominantly in the z-direction aligned field of the circular main magnet 12 is represented by the dashed arrows. The magnet is employed with the field predominantly aligned in the z-direction. The preferred magnetization axis m₀ of the paramagnetic marker 40 aligns with the vertical magnetic field and the paramagnetic marker 40 can freely rotate about the z-axis.

Fig. 14 shows a plot of (x,y)-fluctuations of the paramagnetic marker 40 measured in the twist configuration, i.e. the tethered molecule is arranged with its main axis coinciding with the longitudinal axis of the magnet 12 in a zero point. Since in this situation there is no rotational constraints on the paramagnetic marker from the main magnet, said marker undergoes thermal fluctuations that lie on a circle. This circle is shown in the plot. These thermal fluctuations can be converted to an angle, which can be related to the change in twist of the macromolecule.

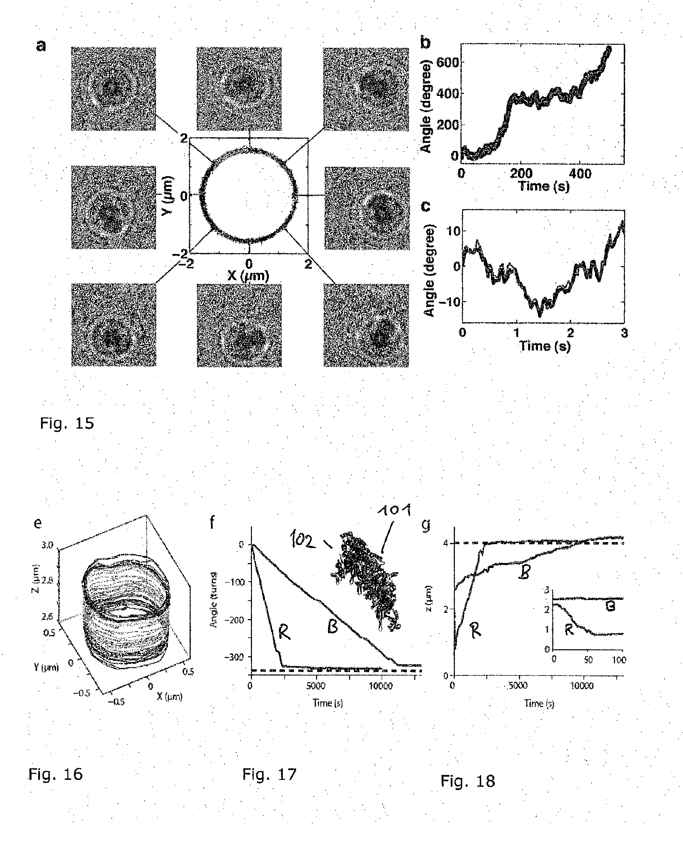

Fig. 15 show the x, y-fluctuations of a tethered 1.4 µm radius paramagnetic marker 40 under the centrally aligned cylindrical magnet 12. A 0.5 µm radius angular tracking marker 42 is attached to the tethered paramagnetic marker 40 and acts as a fiducial marker to detect rotation. All (x, y)-positions are shown in grey. For selected frames from a subsection of the trajectory (shown as the black trace) the corresponding CCD camera images are shown. The paramagnetic marker 40 images were - 4 µm out of focus to facilitate x, y, and z tracking of the position of the paramagnetic marker 40. From the images it is apparent that there is a fixed relationship between the rotation of the bead (traceable from the relative position of the small marker bead) and the position of the paramagnetic marker 40 on the circular annulus in the x, y-plane. The position of the angular tracking marker 42 rotates in synchrony with the position of the paramagnetic marker 40 on the circular annulus, maintaining an angle of -5° anticlockwise from the radial vector. Also shown is a plot of rotation angle of the paramagnetic marker 40 determined from the position of the angular tracking marker 42 (grey) and from the position in the (x, y)-plane (black). Both plots agree so well, that they are indistinguishable. Fig 15(c) shows a zoom of the trace in (b), focusing on the first three seconds. The grey and black traces are clearly distinct, but track each other closely.

Figures 16-18 are an example of the capability of the twist configuration to monitor dynamical changes in the twist of DNA tethers by monitoring the assembly of recombination protein A (RecA) filaments 102 on a 7.9 kbp DNA 101. Upon addition of RecA and the non-hydrolyzable ATP-analogue ATP-γS, paramagnetic markers 40 with a diameter of 1.4 µm held in the twist configuration trace out spiraling trajectories indicating that the DNA tethers lengthen while being unwound (Fig. 16). Grey values indicate progressing time. The spiraling trajectory started at t = 0 s at z ∼ 0 and ended after 1000 s at z ∼µm. Fig. 17 and 18 show how the assembly of the heteroduplex filament to completion could be followed by monitoring rotation angle θ (fig. 17) and height z (fig. 18) as a function of time for a paramagnetic marker 40 with R = 1.4 µm under a pulling force of 6.5 pN (B) and a paramagnetic marker with R = 0.5 µm under a pulling force of 1.5 pN (R). A total unwinding of the 7.9 kbp DNA 101 to -328 ± 10 turns and a lengthening to 4.1 ± 0.1 µm is observed.

## Claims

1. Method for determining one or more characterizing features of a macromolecule (37), said method comprising the steps of:
a) providing a first end of the macromolecule (37) with a paramagnetic marker (40), thereby forming a macromolecule-paramagnetic marker-assembly;
b) tethering at least one other end of the macromolecule (37) of the macromolecule-paramagnetic marker-assembly to one or more tethering points of a holding means (22) for holding said other end of the macromolecule (37), said tethering points being situated in a x,y-plane such that the macromolecule-paramagnetic marker-assembly is arranged substantially in the z-direction perpendicular to the x,y-plane, between a main magnet (12) arranged substantially perpendicular to the x,y-plane and the surface of the holding means (22), wherein the poles (14, 16) of the main magnet (12) are arranged along the z-direction and wherein the main magnet (12) generates a magnetic field directed substantially in the z-direction at the position of the macromolecule-paramagnetic marker-assembly;
c) determining a starting position of the paramagnetic marker (40) of the macromolecule-paramagnetic marker-assembly in a magnetic field generated by the main magnet (12);
d) rotating the main magnet (12) around its magnetic axis, thereby causing the paramagnetic marker (40) to rotate around the magnetic axis of the main magnet (12), to a measuring position;
e) determining the measuring position of the paramagnetic marker (40) of the macromolecule-paramagnetic marker-assembly; and
f) calculating one or more characterizing features from said measuring position;
wherein in step a) the paramagnetic marker (40) is directly attached to the macromolecule (37) and, prior to step c), an auxiliary magnetic field is generated by means of a side magnet (19).

2. Method according to claim 1, wherein the characterizing feature is torque and wherein the paramagnetic marker (40) is provided with an angular tracking marker (42) which allows for the determination of the angular deviation in the position of the paramagnetic marker (40) in the x,y-plane in the measuring position with respect to the starting position in step e).

3. Method according to claim 1 or 2, wherein the side magnet (19) is arranged adjacent to the main magnet (12), in such a way that the poles (14, 16) of the side magnet (19) are opposite to the poles (14, 16) of the main magnet (12).

4. Method according to claim 1, wherein the side magnet (19) is an assembly of electromagnetic coils (51, 52) comprising two spaced apart x-coils (51) that extend in the horizontal x-direction and two spaced apart y-coils (52) that extend in the horizontal y-direction, wherein the electromagnetic coils (51, 52) are powered by current sources (53) through electrical wires (54), wherein the electromagnetic coils (51, 52) are positioned in such a way that the holding means (22) is embedded in the space enclosed by said electromagnetic coils (51, 52).

5. Method according to any of the claims 1 to 4 , wherein the strength ratio of the magnetic force of the main magnet (12) and the magnetic force of the side magnet (19) is within the range of 10000:1 to 10:1.

6. Method according to claim 1, wherein in step b) the tethering point is aligned with the magnetic axis of the main magnet (12) and the characterizing feature is twist.

7. Method according to claim 6, wherein the main magnet (12) and the tethering point of the holding means (22) can be relatively positioned such that the magnetic axis of said main magnet (12) and the tethering point coincide.

8. Method according to claim 7, wherein said magnetic axis of the main magnet (12) and said tethering point coincide within a margin of 0.1 µm.

9. Method according to one of the preceding claims, wherein the paramagnetic marker (40) and/or angular tracking marker (42) of the macromolecule-paramagnetic marker-assembly comprise one or more visualizing elements and wherein the one or more visualizing elements are used as a means to visualize rotation of the macromolecule-paramagnetic marker-assembly.

10. Method according to one of the preceding claims, wherein the macromolecule (37) is a polynucleotide moiety or a polynucleotide moiety complexed with one or more protein moieties.

11. Apparatus (10) for carrying out the method according to one of the preceding claims, said apparatus (10) comprising:
- holding means (22) for holding the macromolecule (37), said holding means (22) comprising one or more tethering points to which at least one end of the macromolecule (37) is tethered, said tethering point being situated in a x,y-plane such that the macromolecule-paramagnetic marker-assembly is arranged substantially in the z-direction perpendicular to the x,y-plane, between a main magnet (12) arranged substantially perpendicular to the x,y-plane and the surface of the holding means (22),;
- a main magnet (12) for generating a magnetic field for applying a force to the macromolecule (37), said main magnet (12) being rotatable around its magnetic axis and arranged at a controllable distance from the holding means (22); the magnetic axis being arranged perpendicular to x,y-plane of the holding means (22), wherein the poles (14, 16) of the main magnet (12) are arranged in the z-direction and wherein the main magnet (12) generates a magnetic field directed substantially in the z-direction at the position of the macro-molecule-paramagnetic marker-assembly;
- means for rotating the main magnet (12); and
- imaging means (32) for imaging information related to the position of the paramagnetic marker (40) and/or an angular tracking marker (42),
which apparatus further comprises a side magnet (19) for allowing the macromolecule (37) provided with a paramagnetic marker (40) to rotate when rotating the main magnet (12),

12. Apparatus (10) according to claim 11, wherein the side magnet (19) is a permanent magnet being attached to a side of the main magnet (12).

13. Apparatus (10) according to claim 12, wherein the side magnet (19) is an assembly of electromagnetic coils (51, 52) comprising two spaced apart x-coils (51) that extend in the horizontal x-direction and two spaced apart y-coils (52) that extend in the horizontal y-direction, wherein the electromagnetic coils (51, 52) are powered by current sources (53) through electrical wires (54), wherein the electromagnetic coils (51, 52) are positioned in such a way that the holding means (22) is embedded in the space enclosed by said electromagnetic coils (51, 52).

14. Apparatus (10) according to any of the claims 11 to 13, wherein the strength ratio of the magnetic force of the main magnet (12) and the magnetic force of the side magnet (19) is within the range of 10000:1 to 10:1.

15. Apparatus (10) according to claim 11, wherein the main magnet (12) and the tethering point of the holding means (22) can be relatively positioned such that the magnetic axis of said main magnet (12) and the tethering point coincide, preferably within a margin of 0,1 µm.

## Patentansprüche

1. Verfahren zur Bestimmung von einem oder mehreren charakteristischen Merkmalen eines Makromoleküls (37), umfassend die folgenden Schritte:
a) Versehen eines ersten Endes des Makromoleküls (37) mit einem paramagnetischen Marker (40) zur Bildung einer Einheit aus Makromolekül und paramagnetischem Marker,
b) Anbinden zumindest eines anderen Endes des Makromoleküls (37) der Einheit aus Makromolekül und paramagnetischem Marker an ein Haltemittel (22) zur Halterung des anderen Endes des Makromoleküls (37), wobei sich die Anbindungspunkte in einer x,y-Ebene befinden, so dass die Einheit aus Makromolekül und paramagnetischem Marker im Wesentlichen in der z-Richtung senkrecht zur x,y-Ebene angeordnet ist zwischen einem Hauptmagneten (12), der im Wesentlichen senkrecht zur x,y-Ebene und der Oberfläche des Haltemittels (22) angeordnet ist, wobei die Pole (14, 16) des Hauptmagneten (12) längs der z-Richtung angeordnet sind und wobei der Hauptmagnet (12) ein magnetisches Feld erzeugt, das im Wesentlichen in die z-Richtung auf die Position der Einheit aus Makromolekül und paramagnetischem Marker gerichtet ist,
c) Bestimmen einer Ausgangsposition des paramagnetischen Markers (40) der Einheit aus Makromolekül und paramagnetischem Marker in einem von dem Hauptmagneten (12) erzeugten Magnetfeld,
d) Drehen des Hauptmagneten (12) um seine Magnetachse, wodurch der paramagnetische Marker (40) um die Magnetachse des Hauptmagneten (12) in eine Messposition gedreht wird,
e) Bestimmen der Messposition des paramagnetischen Markers (40) der Einheit aus Makromolekül und paramagnetischem Marker, und
f) Berechnen von einem oder mehreren charakteristischen Merkmalen aus der Messposition, wobei in Schritt a) der paramagnetische Marker (40) direkt mit dem Makromolekül (37) verbunden ist und vor Schritt c) ein zusätzliches Magnetfeld mit Hilfe eines Seitenmagneten (19) erzeugt wird.

2. Verfahren gemäß Anspruch 1, wobei es sich bei dem charakteristischen Merkmal um das Drehmoment handelt und wobei der paramagnetische Marker (40) mit einem Winkel-Tracking-Marker (42) versehen ist, der die Bestimmung des Winkelfehlers in der Position des paramagnetischen Markers (40) in der x,y-Ebene in der Messposition relativ zu der Ausgangsposition in Schritt e) erlaubt.

3. Verfahren gemäß Anspruch 1 oder 2, wobei der Seitenmagnet (19) benachbart zu dem Hauptmagneten (12) angeordnet ist, so dass die Pole (14, 16) des Seitenmagneten (19) den Polen (14, 16) des Hauptmagneten (12) gegenüberliegen.

4. Verfahren gemäß Anspruch 1, wobei es sich bei dem Seitenmagneten (19) um eine Anordnung von elektromagnetischen Spulen (51, 52) handelt, umfassend zwei voneinander beabstandete x-Spulen (51), die sich in die horizontale x-Richtung erstrecken, sowie zwei voneinander beabstandete y-Spulen (52), die sich in die horizontale y-Richtung erstrecken, wobei die elektromagnetischen Spulen (51, 52) von Stromquellen (53) über elektrische Kabel (54) mit Strom versorgt werden,
wobei die elektromagnetischen Spulen (51, 52) so positioniert sind, dass das Haltemittel (22) in dem von den elektromagnetischen Spulen (51, 52) umgebenen Raum eingebettet ist.

5. Verfahren gemäß einem der Ansprüche 1 bis 4, wobei das Stärkenverhältnis der Magnetkraft des Hauptmagneten (12) zu der Magnetkraft des Seitenmagneten (19) im Bereich von 10000:1 1 bis 10:1 1 liegt.

6. Verfahren gemäß Anspruch 1, wobei der Anbindungspunkt in Schritt b) mit der Magnetachse des Hauptmagneten (12) fluchtet und es sich bei dem charakteristischen Merkmal um den Drall handelt.

7. Verfahren gemäß Anspruch 6, wobei der Hauptmagnet (12) und der Anbindungspunkt des Haltemittels (22) so relativ zueinander positioniert werden können, dass die Magnetachse des Hauptmagneten (12) mit dem Anbindungspunkt zusammenfällt.

8. Verfahren gemäß Anspruch 7, wobei die Magnetachse des Hauptmagneten (12) und der Anbindungspunkt in einem Toleranzbereich von 0,1 im zusammenfallen.

9. Verfahren gemäß einem der vorangehenden Ansprüche, wobei der paramagnetische Marker (40) und/oder der Winkel-Tracking-Marker (42) der Einheit aus Makromolekül und paramagnetischem Marker eines oder mehrere Darstellungselemente umfassen, und wobei das eine oder die mehreren Darstellungselemente als ein Mittel zur Darstellung der Drehung der Einheit aus Makromolekül und paramagnetischem Marker genutzt werden.

10. Verfahren gemäß einem der vorangehenden Ansprüche, wobei es sich bei dem Makromolekül (37) um einen Polynukleotid-Rest oder um einen Polynukleotid-Rest handelt, der mit einem oder mehreren Protein-Resten komplexiert ist.

11. Vorrichtung (10) zur Durchführung des Verfahrens gemäß einem der vorangehenden Ansprüche, umfassend:
- Haltemittel (22) zur Halterung des Makromoleküls (37), wobei das Haltemittel (22) einen oder mehrere Anbindungspunkte umfasst, an den zumindest ein Ende des Makromoleküls (37) angebunden ist, wobei sich der Anbindungspunkt in einer x,y-Ebene befindet, so dass die Einheit aus Makromolekül und paramagnetischem Marker im Wesentlichen in der z-Richtung senkrecht zur x,y-Ebene zwischen einem Hauptmagneten (12) sowie der Oberfläche des Haltemittels (22) angeordnet ist, wobei der Hauptmagnet (12) im Wesentlichen senkrecht zur x,y-Ebene angeordnet ist,
- einen Hauptmagneten (12) zur Erzeugung eines Magnetfeldes zur Ausübung einer Kraft auf das Makromolekül (37), wobei der Hauptmagnet (12) um seine Magnetachse drehbar ist und unter einem einstellbaren Abstand von dem Haltemittel (22) angeordnet ist, wobei die Magnetachse senkrecht zur x,y-Ebene des Haltemittels (22) angeordnet ist, wobei die Pole (14, 16) des Hauptmagneten (12) in der z-Richtung angeordnet sind und wobei der Hauptmagnet (12) ein Magnetfeld erzeugt, das im Wesentlichen in die z-Richtung auf die Position der Einheit aus Makromolekül und paramagnetischem Marker gerichtet ist,
- Mittel zur Drehung des Hauptmagneten (12) und
- Abbildungsmittel (32) zur Abbildung von Informationen bezüglich der Position des paramagnetischen Markers (40) und/oder eines Winkel-Tracking-Markers (42),
wobei die Vorrichtung ferner einen Seitenmagneten (19) umfasst, der bei einer Drehung des Hauptmagneten (12) eine Drehung des mit einem paramagnetischen Marker (40) versehenen Makromoleküls (37) zulässt.

12. Vorrichtung (10) gemäß Anspruch 11, wobei es sich bei dem Seitenmagneten (19) um einen Dauermagneten handelt, der an einer Seite des Hauptmagneten (12) angebracht ist.

13. Vorrichtung (10) gemäß Anspruch 12, wobei es sich bei dem Seitenmagneten (19) um eine Anordnung von elektromagnetischen Spulen (51, 52) handelt, umfassend zwei voneinander beabstandete x-Spulen (51), die sich in die horizontale x-Richtung erstrecken, sowie zwei voneinander beabstandete y-Spulen (52), die sich in die horizontale yRichtung erstrecken, wobei die elektromagnetischen Spulen (51, 52) von Stromquellen (53) über elektrische Leitungen (54) mit Strom versorgt werden, wobei die elektromagnetischen Spulen (51, 52) so positioniert sind, dass das Haltemittel (22) in dem von den elektromagnetischen Spulen (51, 52) umschlossenen Raum eingebettet ist.

14. Vorrichtung (10) gemäß einem der Ansprüche 11 bis 13, wobei das Stärkenverhältnis der Magnetkraft des Hauptmagneten (12) zu der Magnetkraft des Seitenmagneten (19) im Bereich von 10000:1 bis10:1 liegt.

15. Vorrichtung (10) gemäß Anspruch 11, wobei der Hauptmagnet (12) und der Anbindungspunkt des Haltemittels (22) so relativ zueinander positioniert werden können, dass die Magnetachse des Hauptmagneten (12) und der Anbindungspunkt zusammenfallen, vorzugsweise in einem Toleranzbereich von 0,1 µm.

## Revendications

1. Procédé destiné à déterminer une ou plusieurs caractéristiques d'une macromolécule (37), ledit procédé comprenant les étapes consistant à :
a) doter une première extrémité de la macromolécule (37) d'un marqueur paramagnétique (40), formant ainsi un ensemble macromolécule/marqueur paramagnétique ;
b) attacher au moins une autre extrémité de la macromolécule (37) de l'ensemble macromolécule/marqueur paramagnétique à un ou plusieurs points d'attache d'un moyen de maintien (22) destiné à maintenir ladite autre extrémité de la macromolécule (37), lesdits points d'attache étant situés dans un plan x,y de telle sorte que l'ensemble macromolécule/marqueur paramagnétique est disposé sensiblement dans la direction z perpendiculaire au plan x,y, entre un aimant principal (12) disposé sensiblement perpendiculairement au plan x,y et la surface du moyen de maintien (22), dans lequel les pôles (14 , 16) de l'aimant principal (12) sont disposés le long de la direction z, et dans lequel l'aimant principal (12) génère un champ magnétique dirigé sensiblement dans la direction z à la position de l'ensemble macromolécule/marqueur paramagnétique ;
c) déterminer une position de départ du marqueur paramagnétique (40) de l'ensemble macromolécule/marqueur paramagnétique dans un champ magnétique généré par l'aimant principal (12) ;
d) faire tourner l'aimant principal (12) autour de son axe magnétique, provoquant ainsi la rotation du marqueur paramagnétique (40) autour de l'axe magnétique de l'aimant principal (12) vers une position de mesure ;
e) déterminer la position de mesure du marqueur paramagnétique (40) de l'ensemble macromolécule/marqueur paramagnétique ; et
f) calculer une ou plusieurs caractéristiques à partir de ladite position de mesure ;
et dans lequel à l'étape a) le marqueur paramagnétique (40) est directement attaché à la macromolécule (37) et, avant l'étape c), un champ magnétique auxiliaire est généré au moyen d'un aimant latéral (19).

2. Procédé selon la revendication 1, dans lequel la caractéristique est le couple et dans lequel le marqueur paramagnétique (40) est doté d'un marqueur de localisation angulaire (42) qui permet de déterminer l'écart angulaire de la position du marqueur paramagnétique (40) dans le plan x,y dans la position de mesure par rapport à la position de départ à l'étape e).

3. Procédé selon la revendication 1 ou 2, dans lequel l'aimant secondaire (19) est disposé au voisinage de l'aimant principal (12), de telle manière que les pôles (14, 16) de l'aimant latéral (19) sont opposés aux pôles (14, 16) de l'aimant principal (12).

4. Procédé selon la revendication 1, dans lequel l'aimant latéral (19) est un ensemble de bobines électromagnétiques (51, 52) comprenant deux bobines x (51) espacées l'une de l'autre qui s'étendent dans la direction horizontale x et deux bobines y (52) espacées l'une de l'autre qui s'étendent dans la direction horizontale y, dans lequel les bobines électromagnétiques (51, 52) sont alimentées par des sources de courant (53) par le biais de fils électriques (54), dans lequel les bobines électromagnétiques (51, 52) sont positionnées de telle manière que le moyen de maintien (22) est incorporé dans l'espace entouré par lesdites bobines électromagnétiques (51, 52).

5. Procédé selon l'une quelconque des revendications 1 à 4, dans lequel le rapport de puissance de la force magnétique de l'aimant principal (12) et de la force magnétique de l'aimant latéral (19) est dans la gamme de 10000:1 à 10:1.

6. Procédé selon la revendication 1, dans lequel à l'étape b) le point d'attache est aligné avec l'axe magnétique de l'aimant principal (12) et la caractéristique est la torsion.

7. Procédé selon la revendication 6, dans lequel l'aimant principal (12) et le point d'attache du moyen de maintien (22) peuvent être positionnés de manière relative de telle sorte que l'axe magnétique de l'aimant principal (12) et le point d'attache coïncident.

8. Procédé selon la revendication 7, dans lequel ledit axe magnétique de l'aimant principal (12) et ledit point d'attache coïncident dans une marge de 0,1 µm.

9. Procédé selon l'une des revendications précédentes, dans lequel le marqueur paramagnétique (40) et/ou un marqueur de localisation angulaire (42) de l'ensemble macromolécule/marqueur paramagnétique comprennent un ou plusieurs éléments de visualisation et dans lequel un ou plusieurs éléments de visualisation sont utilisés comme moyen pour visualiser la rotation de l'ensemble macromolécule/marqueur paramagnétique.

10. Procédé selon l'une des revendications précédentes, dans lequel la macromolécule (37) est un fragment de polynucléotide ou un fragment de polynucléotide complexé avec un ou plusieurs fragments de protéines.

11. Appareil (10) destiné à mettre en oeuvre le procédé selon l'une quelconque des revendications précédentes, ledit appareil (10) comprenant :
- un moyen de maintien (22) destiné à maintenir la macromolécule (37), ledit moyen de maintien (22) comprenant un ou plusieurs points d'attache auxquels au moins une extrémité de la macromolécule (37) est attachée, ledit point d'attache étant situé dans un plan x,y de telle sorte que l'ensemble macromolécule/marqueur paramagnétique est disposé sensiblement dans la direction z perpendiculaire au plan x,y, entre un aimant principal (12) disposé sensiblement perpendiculairement au plan x,y et la surface du moyen de maintien (22) ;
- un aimant principal (12) destiné à générer un champ magnétique pour appliquer une force à la macromolécule (37), ledit aimant principal (12) pouvant tourner autour de son axe magnétique et étant disposé à une distance réglable du moyen de maintien (22) ; les axes magnétiques étant disposés perpendiculairement au plan x,y du moyen de maintien (22), dans lequel les pôles (14, 16) de l'aimant principal (12) sont disposés dans la direction z, et dans lequel l'aimant principal (12) génère un champ magnétique dirigé sensiblement dans la direction z à la position de l'ensemble macromolécule/marqueur paramagnétique ;
- un moyen pour faire tourner l'aimant principal (12) ; et
- un moyen d'imagerie (32) permettant d'obtenir une image des informations relatives à la position du marqueur paramagnétique (40) et/ou d'un marqueur de localisation angulaire (42),
lequel appareil comprend en outre un aimant latéral (19) destiné à permettre à la macromolécule (37) doté d'un marqueur paramagnétique (40) de tourner lors de la rotation de l'aimant principal (12).

12. Appareil (10) selon la revendication 11, dans lequel l'aimant latéral (19) est un aimant permanent attaché à un côté de l'aimant principal (12).

13. Appareil (10) selon la revendication 12, dans lequel l'aimant secondaire (19) est un ensemble de bobines électromagnétiques (51, 52) comprenant deux bobines x (51) espacées l'une de l'autre qui s'étendent dans la direction horizontales x et deux bobines y (52) espacées l'une de l'autre qui s'étendent dans la direction horizontale y, dans lequel les bobines électromagnétiques (51, 52) sont alimentées par des sources de courant (53) par le biais de fils électriques (54), dans lequel les bobines électromagnétiques (51, 52) sont positionnées de telle manière à ce que le moyen de maintien (22) est incorporé dans l'espace entouré par lesdites bobines électromagnétiques (51, 52).

14. Dispositif (10) selon l'une quelconque des revendications 11 à 13, dans lequel le rapport de puissance de la force magnétique de l'aimant principal (12) et de la force magnétique de l'aimant latéral (19) est dans la gamme de 10000:1 à 10:1.

15. Dispositif (10) selon la revendication 11, dans lequel l'aimant principal (12) et le point d'attache du moyen de maintien (22) peuvent être positionnés de manière relative de telle sorte que l'axe magnétique de l'aimant principal (12) et le point d'attache coïncident, de préférence dans une marge de 0,1 µm.
